# EUROPEAN PATENT SPECIFICATION

(11) **EP 3 856 048 B1**
(45) Date of publication and mention of the grant of the patent: **09.04.2025**
(21) Application number: 19787126.2
(22) Date of filing: 24.09.2019
(51) Int. Cl.: A61B 17/16, A61B 17/3207

(54) **CHONDROTOME**
CHONDROTOM
CHONDROTOME

(30) Priority: 24.09.2018 US 201862735290 P
(43) Date of publication of application: 04.08.2021
(73) Proprietor: CONMED Corporation, Largo, FL 33773 (US)
(72) Inventor: VIOLA, Randall, Vail, CO 81657 (US); ALFONSO, Gregory, Seffner, FL 33584 (US); SUMMITT, Matthew, Palm Harbor, FL 34683 (US); THIBODEAU, Robert, Saint Petersburg, FL 33710 (US)
(74) Representative: Strehl & Partner mbB
(86) International application number: PCT/US2019/052550
(87) International publication number: WO 2020/068718

(56) References cited:
- DE-C- 865 784
- FR-A1- 2 937 239
- US-A- 5 586 989
- US-A1- 2010 191 173
- US-A1- 2017 056 029

## Description

### CROSS-REFERENCE TO RELATED APPLICATIONS

This application claims priority to U.S. Provisional Patent Application Serial No. 62/735,290, filed on September 24, 2018 and entitled "Chondrotome".

### BACKGROUND OF THE INVENTION

### 1. Field of the Invention

The present invention is directed generally to a surgical cutting device and, more particularly, to surgical cutting device with a blade for cutting and removing cartilage from a diseased area.

### 2. Description of Related Art

A variety of conditions may negatively affect the human knee including arthritis, ligament instability, and localized trauma. These conditions may be surgically treated by repairing portions of the knee cartilage, meniscus, or ligaments or by replacing them with natural or artificial replacements. In such procedures, it is often necessary to correct the alignment of the knee to relieve pressure from the damaged portion of the joint and balance the load on the joint. In some instances, correcting the knee alignment alone, without additional repair or replacement of injured or diseased tissue, is sufficient to provide relief and improve function. Often, in a young arthritis patient, correcting the knee alignment may be a first surgical choice to provide years of relief prior to resorting to a more aggressive total knee replacement procedure. Typically, injured or diseased knees will develop a varus deformity in which the medial side of the person's knee joint has become compressed resulting in a bowlegged alignment of the lower limb.

A frequently used procedure to correct knee alignment is high tibial osteotomy. In this procedure, the knee alignment is changed by cutting the tibia on one side and then expanding or compressing the cut side to change the angle between the axes of the tibia and femur. For example, in a closing high tibial osteotomy, a wedge of bone is removed from the tibia and then the opposite sides of the cut are brought together to angle the tibia towards the side on which the cut was made. In an opening high tibial osteotomy, a cut is made partway across the tibia and the cut is opened to create a wedge-shaped gap thereby angling the tibia away from the side on which the cut was made. A fixturing device, such as a bone plate or external fixator, is then applied to the tibia to hold the tibial alignment until the bone heals. Typically, for opening high tibial osteotomy, bone is taken from the patient's pelvis and applied to the gap in the tibia to aid in bone healing. An opening high tibial osteotomy may be performed on the medial side of a patient's knee to treat a varus deformity.

Another procedure used to correct knee alignment is a distal femoral osteotomy. For example, in an opening lateral distal femoral osteotomy, a cut is made partway across the femur from the lateral side and the cut is opened to create a wedge-shaped gap thereby angling the femur away from the lateral side.

It is often necessary to use powered tissue cutting tools in order to perform surgical procedures. Such tools generally comprise a handpiece which cyclically moves a tissue cutting device such as a blade or burr in some oscillating or reciprocating manner. The handpiece generally includes a pneumatic or electric drive motor having an output shaft to which the cutting device is attached, the shaft being axially aligned with a drive axis of the handpiece. As used herein, the term "drive axis" refers to the axis of the motor output shaft through which power is delivered from the motor. The handpiece may be a "pencil" type handpiece in which the body is elongated and the drive axis is aligned with the axis of the body or a pistol-grip type of handpiece in which the drive axis is aligned in a chosen direction relative to the grip. The drive motor of the handpiece produces a driving force which reciprocates the output shaft and cutting device either longitudinally, i.e. linearly along the drive axis (like a saber saw), or arcuately in a plane perpendicular to the drive axis. Handpieces utilizing the former type of action are generally referred to as reciprocating saws while those utilizing the latter action are generally referred to as oscillating saws. In some cases an oscillating saw may transfer the oscillating drive motion so that it is cyclical within a plane parallel to the axis of the elongated body of the handpiece. A sagittal saw is a type of oscillatory saw in which the cyclical reciprocating action is in a plane aligned with the drive axis.

In all instances, numerous conventional tissue cutting blades or burrs or other devices (all collectively referred to herein as "blades") are adapted to be secured to the handpiece via a collet mechanism which is utilized to selectively attach and release a desired blade. A variety of different cuts can be made with a single saw depending upon the shape of the blade. For oscillating saws, the blades are often in the form of a flat, elongated body having a cutting edge (e.g. teeth, abrader, etc.) at one end and a hub at the other end, the hub being shaped and adapted to fit the particular collet. Such flat blades are used to make cuts in a plane perpendicular to the drive axis. An oscillating saw may also be used for effecting cuts in a plane parallel to the handpiece axis by attaching a transverse hub to a flat blade. A crescentic blade having a curved instead of a flat body may be used for curved cuts. The body of a crescentic blade has teeth (or another cutting edge) at its distal end which follow the shape of the arcuate body so that as the blade oscillates about the axis, the teeth follow an arcuate pattern having a center of curvature on the axis. Some flat blades may have the cutting edge at an angle to the blade body to make cuts in an angled plane. Angled blades may have an axially elongated body either on or off the blade axis. That is, the proximal end of the blade body may have a transverse hub or it may simply be attached in-line with the drive axis. The resulting cut is arcuate and facing away from the axis.

Any of the blades described above may be used to make cuts into the bone. However, in most surgical procedures involving the knee (or foot, angle, hand and/or wrist), damage or diseased cartilage may also need to be removed from the surgical site for proper repair of a particular surgical site.

Therefore, there is a need for a tool that is specialized for cutting and removing diseased cartilage.

US 2010/191173 A1 discloses a surgical cutting device which forms the basis for the two-part form of present claim 1. Other conventional cutting devices are described in DE 865 784 C, US 5 586 989 A, FR 2 937 239 A1 and US 2017/056029 A1.

### SUMMARY OF THE INVENTION

Embodiments of the present invention are directed to a surgical cutting device for cutting and removing cartilage from a diseased area. In particular, embodiments of the present invention are used to cut and scrape away diseased or damaged cartilage (e.g., in extremities procedures (foot and ankle / hand and wrist). According to one aspect, the surgical cutting device includes an elongated shaft having a proximal portion and a distal narrowing portion connected between a proximal end and a distal end. A central longitudinal axis extends through the elongated shaft. The proximal portion has a first diameter and the distal narrowing portion has a second diameter. The first diameter is larger than the second diameter. The cutting device also includes a cutting tip at the distal end of the elongated shaft. The cutting tip extends at an angle relative to the central longitudinal axis and includes a curved blade.

According to another aspect, the surgical cutting device includes an elongated shaft having a proximal portion and a distal narrowing portion connected between a proximal end and a distal end. A central longitudinal first axis extends through the elongated shaft. The surgical cutting device also includes a cutting tip at the distal end of the elongated shaft. The cutting tip extends along a second axis, which is at an angle relative to the central longitudinal first axis. The cutting tip comprises a curved blade which is open in a proximal direction.

The surgical cutting device can also include a handle attached at the proximal end of the elongated shaft.

These and other aspects of the invention will be apparent from and elucidated with reference to the embodiment(s) described hereinafter.

### BRIEF DESCRIPTION OF THE DRAWINGS

One or more aspects of the present invention are particularly pointed out and distinctly claimed in the claims at the conclusion of the specification. The foregoing and other objects, features, and advantages of the invention are apparent from the following description taken in conjunction with the accompanying drawings in which:
FIG. 1 is a side perspective view schematic representation of a cutting device, according to an embodiment;
FIG. 2 is a close-up, side perspective view schematic representation of the distal end of the cutting device, according to an embodiment;
FIG. 3 is a side view schematic representation of a cutting device, according to an alternative embodiment; and
FIG. 4 is a close-up, top perspective view schematic representation of the distal end of the cutting device, according to an alternative embodiment.

### DETAILED DESCRIPTION OF THE INVENTION

Aspects of the present invention and certain features, advantages, and details thereof, are explained more fully below with reference to the non-limiting examples illustrated in the accompanying drawings. Descriptions of well-known structures are omitted so as not to unnecessarily obscure the invention in detail. It should be understood, however, that the detailed description and the specific non-limiting examples, while indicating aspects of the invention, are given by way of illustration only, and are not by way of limitation. Various substitutions, modifications, additions, and/or arrangements, within the scope of the claims will be apparent to those skilled in the art from this disclosure.

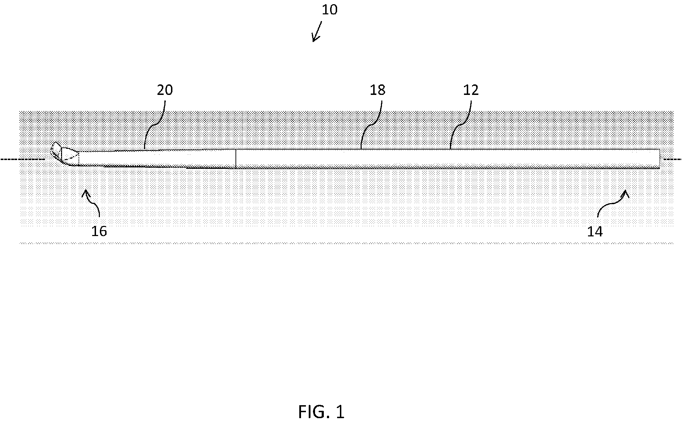

Referring now to the figures, wherein like reference numerals refer to like parts throughout, FIG. 1 shows a side perspective view schematic representation of a cutting device 10, according to an embodiment. The cutting device 10 comprises an elongated shaft 12 extending along a central longitudinal y - y axis between a proximal end 14 and a distal end 16. In the depicted embodiment, the elongated shaft 12 is cylindrical; however, any other suitable geometry may be used. The elongated shaft 12 may have a uniform diameter or a varied diameter. In the depicted embodiment, the elongated shaft 12 has a first diameter and a second diameter that is different from the first diameter.

As shown in FIG. 1, the elongated shaft 12 has proximal portion 18 and a distal portion 20 (which can be narrowing and taper in the distal direction, but does not need to be). The proximal portion 18 has the first diameter and the distal narrowing portion 20 has the second diameter. In the depicted embodiment, the second diameter of the distal narrowing portion 20 is smaller than the first diameter of the proximal portion 18. In an embodiment, the distal narrowing portion 20 can be tapered in the distal direction and the second diameter is any diameter along the distal narrowing portion 20. The narrowing portion 20 allows the cutting device 10 to access and cut smaller surgical areas, such as in the hand and wrist.

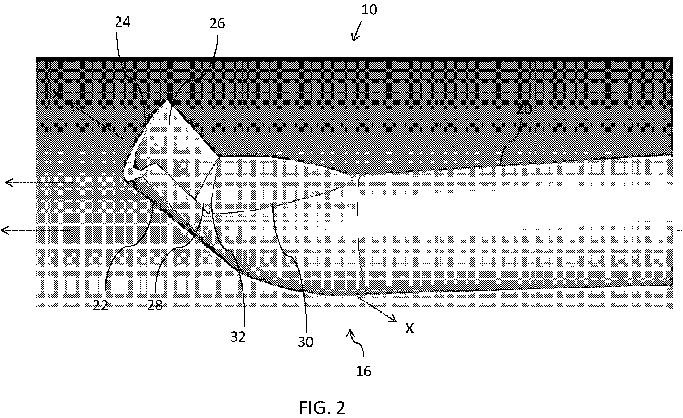

Turning now to FIG. 2, there is shown a close-up, side perspective view schematic representation of the distal end 16 of the cutting device 10, according to an embodiment. The narrowing portion 20 of the elongated shaft 12 extends to the distal end 16. The distal end 16 comprises a cutting tip 22, as shown in FIG. 2. In the depicted embodiment, the cutting tip 22 extends along an x - x axis. In general, the cutting tip 22 extends at angle relative to the central longitudinal y - y axis (which extends through the elongated shaft 12). In FIG. 2, the cutting tip 22 extends at an angle greater than 90 degrees relative to the central longitudinal y - y axis.

Still referring to FIG. 2, the cutting tip 22 comprises a curved blade 24. In the depicted embodiment, the curved blade 24 has a semi-circular cross-section such that the curved blade 24 is crescent-shaped and open toward the proximal direction. The curved blade 24 comprises an inner wall 26 extending to a flat, semi-circular base 28. Further, the base 28 of the curved blade 24 extends to and abuts a flat portion 30 of the cutting tip 22. In the depicted embodiment, the flat portion 30 is D-shaped and extends proximally.

In the embodiment depicted in FIG. 2, the base 28 of the curved blade 24 and the flat portion 30 are connected at a flange 32 or edge. The base 28 extends at an angle relative to the flat portion 30. In an embodiment, the flat portion 30 extends along both a plane and an axis (z - z axis) that are substantially parallel to the central longitudinal y - y axis, while the base 28 extends along a plane and axis that is transverse to both the central longitudinal y - y axis and the x - x axis. In one embodiment, the base 28 extends along an axis which is substantially perpendicular to the x - x axis. The angular configuration and curvature of the cutting tip 22 is optimized for use in hand and wrist procedures.

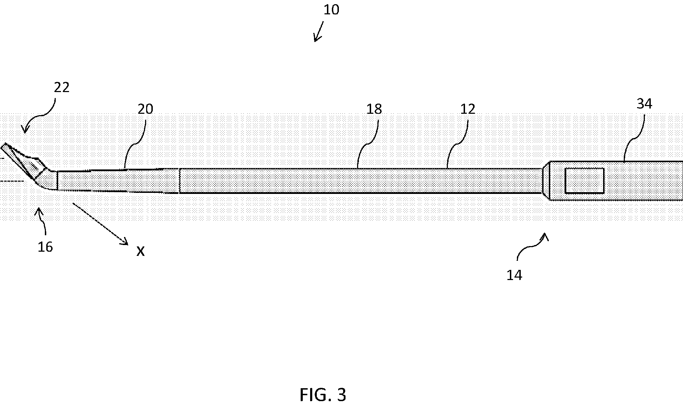

Referring now to FIG. 3, there is shown a side view schematic representation of a cutting device 10, according to an alternative embodiment. In the depicted embodiment, the proximal end 14 of the cutting device 10 comprises a handle 34 . In accordance with an embodiment, there is no motor or automated actuation for the described device 10, which include curved blade(s) or cutting tip on a shaft that will be stuck into a handle 34 so the surgeon can cut and pry and scrape away cartilage. Thus, the cutting device 10 can be manipulated directly by the user via the handle 34 (or otherwise at the proximal end 14 of the device 10).

Similar to the embodiment shown in FIGs. 1 and 2, the embodiment of the cutting device 10 in FIG. 3 comprises an elongated shaft 12 extending along a central longitudinal y - y axis between a proximal end 14 and a distal end 16. The elongated shaft 12 may be cylindrical and have a uniform diameter or a varied diameter, as described above. As also similar to the embodiment shown in FIGs. 1 and 2, the elongated shaft 12 of the cutting device 10 in FIG. 3 comprises a proximal portion 18 and a distal narrowing portion 20. As shown, the distal narrowing portion 20 is tapered in the distal direction. The distal narrowing portion 20 allows the cutting device 10 to access and cut small surgical areas, such as in the foot and ankle.

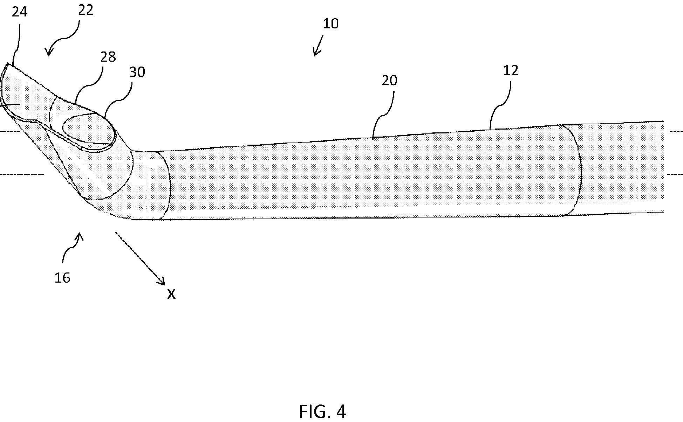

The distal end 16 of the cutting device 10 comprises a cutting tip 22. In the depicted embodiment, the cutting tip 22 extends along an x - x axis. In general, the cutting tip 22 extends at angle relative to the central longitudinal y - y axis (which extends through the elongated shaft 12). In FIG. 3, the cutting tip 22 extends at an angle greater than 90 degrees relative to the central longitudinal y - y axis. The cutting tip 22 in FIG. 3 is configured and optimized for use in foot and ankle procedures. For example, the cutting tip 22 is longer than the cutting tip 22 in the embodiment in FIGs. 1 and 2. Accordingly, the cutting tip 22 of the cutting device 10 in FIGs. 3 and 4 varies from that shown in FIGs. 1 and 2.

Turning now to FIG. 4, there is shown a close-up, side perspective view schematic representation of the distal end 16 of the cutting device 10, according to an alternative embodiment. As shown in FIG. 4, the cutting tip 22 comprises a curved blade 24 which is open in the proximal direction. In the depicted embodiment, the curved blade 24 has a semi-circular cross-section such that the curved blade 24 is crescent-shaped. The curved blade 24 comprises an inner wall 26 extending to a base 28. The base 28 in the embodiment shown in FIG. 3-4 is sloped, extending toward the central longitudinal y - y axis (and elongated shaft 12). Further, the sloped base 28 of the curved blade 24 extends to and abuts a flat portion 30 of the cutting tip 22. In the depicted embodiment, the flat portion 30 is circular or semi-circular and extends proximally.

In the embodiment depicted in FIG. 4, the base 28 of the curved blade 24 slopes or extends to the flat portion 30 of the curved blade 24. The base 28 also extends at least partially around the flat portion 30, as shown in FIG. 4. The flat portion 30 extends along a plane and a z - z axis, which are substantially parallel to the central longitudinal y - y axis, as shown in FIG. 3. The angular configuration and curvature of the cutting tip 22 is optimized for use in foot and ankle procedures.

While various embodiments have been described and illustrated herein, those of ordinary skill in the art will readily envision a variety of other means and/or structures for performing the function and/or obtaining the results and/or one or more of the advantages described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific embodiments described herein. It is, therefore, to be understood that the foregoing embodiments are presented by way of example only and that, within the scope of the appended claims, embodiments may be practiced otherwise than as specifically described and claimed.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as, "has" and "having"), "include" (and any form of include, such as "includes" and "including"), and "contain" (any form of contain, such as "contains" and "containing") are open-ended linking verbs. As a result, an element of a device that "comprises", "has", "includes" or "contains" one or more features possesses those one or more features, but is not limited to possessing only those one or more features. Furthermore, a device or structure that is configured in a certain way is configured in at least that way, but may also be configured in ways that are not listed.

The description of the present invention has been presented for purposes of illustration and description, but is not intended to be exhaustive or limited to the invention in the form disclosed. Many modifications and variations will be apparent to those of ordinary skill in the art without departing from the scope of the invention as defined by the claims. The embodiment was chosen and described in order to best explain the principles of one or more aspects of the invention and the practical application, and to enable others of ordinary skill in the art to understand one or more aspects of the present invention for various embodiments with various modifications as are suited to the particular use contemplated.

## Claims

1. A surgical cutting device (10), comprising:
an elongated shaft (12) having a proximal portion (18) and a distal portion (20) connected between a proximal end (14) and a distal end (16);
a central longitudinal axis (y-y) extending through the elongated shaft (12);
wherein the proximal portion (18) has a first diameter and the distal portion (20) has a second diameter, the first diameter being larger than the second diameter;
a cutting tip (22) at the distal end (16) of the elongated shaft (12), extending along a second axis (x-x), at an angle relative to the central longitudinal axis (y-y);
wherein the cutting tip (22) comprises a curved blade (24) which is open in a proximal direction;
wherein the curved blade (24) comprises an inner wall (26) extending to a base (28) of the cutting tip (22),
**characterized in that** a cross-section of the curved blade (24) in a plane perpendicular to the second axis (x-x) is crescent-shaped, and the base (28) is semi-circular.

2. The device (10) of claim 1, wherein the distal portion (20) is tapered, decreasing in diameter in a distal direction.

3. The device (10) of claim 1, wherein the angle at which the cutting tip (22) extends relative to the central longitudinal axis (y-y) is greater than 90 degrees.

4. The device (10) of claim 1, further comprising a flat portion (30) on the cutting tip (22), wherein the flat portion (30) is connected to the curved blade (24) and extends in a proximal direction.

5. The device (10) of claim 4, wherein the flat portion (30) extends along a plane which is substantially parallel to the central longitudinal axis (y-y).

6. The device (10) of claim 1, wherein the base (28) extends along a plane which is substantially perpendicular to the second axis (x-x).

7. The device (10) of claim 1, further comprising a flat portion (30) connected to the base (28) of the cutting tip (22) by a flange (32).

8. The device (10) of claim 7, wherein the flat portion (30) is D-shaped.

9. The device (10) of claim 1, further comprising a handle (34) attached at the proximal end (14) of the elongated shaft (12).

10. The device (10) of claim 9, wherein the base (28) is sloped toward the elongated shaft (12).

11. The device (10) of claim 9, further comprising a flat portion, wherein the base (28) extends to and at least partially around a flat portion (30) of the cutting tip (22).

12. The device (10) of claim 11, wherein the flat portion (30) is circular or semi-circular.

13. The device of claim 7 or 11, wherein the flat portion (30) extends in a proximal direction along a third axis (z-z) which is substantially parallel to the central longitudinal first axis (y-y).

## Patentansprüche

1. Chirurgische Schneidvorrichtung (10), umfassend:
einen länglichen Schaft (12) mit einem proximalen Abschnitt (18) und einem distalen Abschnitt (20), die zwischen einem proximalen Ende (14) und einem distalen Ende (16) verbunden sind;
eine zentrale Längsachse (y-y), die durch den länglichen Schaft (12) verläuft;
wobei der proximale Abschnitt (18) einen ersten Durchmesser und der distale Abschnitt (20) einen zweiten Durchmesser aufweist, wobei der erste Durchmesser größer als der zweite Durchmesser ist;
eine Schneidspitze (22) an dem distalen Ende (16) des länglichen Schafts (12), die sich entlang einer zweiten Achse (x-x) unter einem Winkel relativ zur zentralen Längsachse (y-y) erstreckt;
wobei die Schneidspitze (22) eine gebogene Klinge (24) umfasst, die in einer proximalen Richtung offen ist;
wobei die gebogene Klinge (24) eine Innenwand (26) umfasst, die sich zu einer Basis (28) der Schneidspitze (22) erstreckt,
**dadurch gekennzeichnet, dass** ein Querschnitt der gebogenen Klinge (24) in einer Ebene senkrecht zur zweiten Achse (x-x) sichelförmig ist und die Basis (28) halbkreisförmig ist.

2. Vorrichtung (10) nach Anspruch 1, wobei sich der distale Abschnitt (20) verjüngt und sein Durchmesser in einer distalen Richtung abnimmt.

3. Vorrichtung (10) nach Anspruch 1, wobei der Winkel, in dem sich die Schneidspitze (22) relativ zur zentralen Längsachse (y-y) erstreckt, größer als 90 Grad ist.

4. Vorrichtung (10) nach Anspruch 1, ferner umfassend einen flachen Abschnitt (30) an der Schneidspitze (22), wobei der flache Abschnitt (30) mit der gebogenen Klinge (24) verbunden ist und sich in eine proximale Richtung erstreckt.

5. Vorrichtung (10) nach Anspruch 4, wobei sich der flache Abschnitt (30) entlang einer Ebene erstreckt, die im Wesentlichen parallel zu der zentralen Längsachse (y-y) liegt.

6. Vorrichtung (10) nach Anspruch 1, wobei sich die Basis (28) entlang einer Ebene erstreckt, die im Wesentlichen senkrecht zur zweiten Achse (x-x) ist.

7. Vorrichtung (10) nach Anspruch 1, ferner umfassend einen flachen Abschnitt (30), der mit der Basis (28) der Schneidspitze (22) durch einen Flansch (32) verbunden ist.

8. Vorrichtung (10) nach Anspruch 7, wobei der flache Abschnitt (30) D-förmig ist.

9. Vorrichtung (10) nach Anspruch 1, ferner umfassend einen Griff (34), der am proximalen Ende (14) des länglichen Schafts (12) angebracht ist.

10. Vorrichtung (10) nach Anspruch 9, wobei die Basis (28) in Richtung des länglichen Schafts (12) geneigt ist.

11. Vorrichtung (10) nach Anspruch 9, ferner umfassend einen flachen Abschnitt, wobei sich die Basis (28) bis zum flachen Abschnitt (30) der Schneidspitze (22) und zumindest teilweise um diesen herum erstreckt.

12. Vorrichtung (10) nach Anspruch 11, wobei der flache Abschnitt (30) kreisförmig oder halbkreisförmig ist.

13. Vorrichtung nach Anspruch 7 oder 11, wobei sich der flache Abschnitt (30) in einer proximalen Richtung entlang einer dritten Achse (z-z) erstreckt, die im Wesentlichen parallel zur zentralen Längsachse (y-y) ist.

## Revendications

1. Dispositif chirurgical (10) de coupe, comprenant :
un arbre allongé (12) ayant une partie proximale (18) et une partie distale (20) connectées entre une extrémité proximale (14) et une extrémité distale (16) ;
un axe longitudinal central (y-y) s'étendant à travers l'arbre allongé (12) ;
dans lequel la partie proximale (18) a un premier diamètre et la partie distale (20) a un deuxième diamètre, le premier diamètre étant plus grand que le deuxième diamètre ;
une pointe (22) de coupe à l'extrémité distale (16) de l'arbre allongé (12), s'étendant le long d'un deuxième axe (x-x), à un angle par rapport à l'axe longitudinal central (y-y) ;
dans lequel la pointe (22) de coupe comprend une lame incurvée (24) qui est ouverte dans un sens proximal ;
dans lequel la lame incurvée (24) comprend une paroi intérieure (26) s'étendant jusqu'à une base (28) de la pointe (22) de coupe,
**caractérisé en ce qu'**une coupe transversale de la lame incurvée (24) dans un plan perpendiculaire au deuxième axe (x-x) est en forme de croissant, et la base (28) est semi-circulaire.

2. Dispositif (10) selon la revendication 1, dans lequel la partie distale (20) est conique, diminuant en diamètre dans un sens distal.

3. Dispositif (10) selon la revendication 1, dans lequel l'angle auquel la pointe (22) de coupe s'étend par rapport à l'axe longitudinal central (y-y) est supérieur à 90 degrés.

4. Dispositif (10) selon la revendication 1, comprenant en outre une partie plate (30) sur la pointe (22) de coupe, dans lequel la partie plate (30) est connectée à la lame incurvée (24) et s'étend dans un sens proximal.

5. Dispositif (10) selon la revendication 4, dans lequel la partie plate (30) s'étend le long d'un plan qui est sensiblement parallèle à l'axe longitudinal central (y-y).

6. Dispositif (10) selon la revendication 1, dans lequel la base (28) s'étend le long d'un plan qui est sensiblement perpendiculaire au deuxième axe (x-x).

7. Dispositif (10) selon la revendication 1, comprenant en outre une partie plate (30) connectée à la base (28) de la pointe (22) de coupe par une bride (32).

8. Dispositif (10) selon la revendication 7, dans lequel la partie plate (30) est en forme de D.

9. Dispositif (10) selon la revendication 1, comprenant en outre une poignée (34) fixée à l'extrémité proximale (14) de l'arbre allongé (12).

10. Dispositif (10) selon la revendication 9, dans lequel la base (28) est en pente vers l'arbre allongé (12).

11. Dispositif (10) selon la revendication 9, comprenant en outre une partie plate, dans lequel la base (28) s'étend jusqu'à et au moins partiellement autour d'une partie plate (30) de la pointe (22) de coupe.

12. Dispositif (10) selon la revendication 11, dans lequel la partie plate (30) est circulaire ou semi-circulaire.

13. Dispositif (10) selon la revendication 7 ou 11, dans lequel la partie plate (30) s'étend dans un sens proximal le long d'un troisième axe (z-z) qui est sensiblement parallèle au premier axe longitudinal central (y-y).
